(19) Europäisches Patentamt
European Patent Office
Office européen des brevets

(11) Veröffentlichungsnummer: **0 000 207**
**A1**

# (12) EUROPÄISCHE PATENTANMELDUNG

(21) Anmeldenummer: 78100303.3

(22) Anmeldetag: 04.07.78

(51) Int. Cl.²: C 07 C 177/00

(30) Priorität: 05.07.77 US 813068

(43) Veröffentlichungstag der Anmeldung: 10.01.79 Patentblatt 79/01

(84) Benannte Vertragsstaaten: BE CH DE FR GB LU NL SE

(71) Anmelder: F. Hoffmann-La Roche & Co. Aktiengesellschaft, Abt. VIII-Pt, CH-4002 Basel (CH)

(72) Erfinder: Holland, George William, 10 Acorn Place, North Caldwell, N.J. (US)

(72) Erfinder: Rosen, Perry, 26 Sunset Drive, North Caldwell, N.J. (US)

(74) Vertreter: Lederer, Franz, Dr. et al, Patentanwälte Lederer, Meyer Lucile-Grahn-Strasse 22, D-8000 München 80 (DE)

## (54) Neue Prostaglandinderivate, deren Herstellung und pharmazeutische Präparate und Zwischenprodukte.

(57) Prostaglandinderivate der Formel

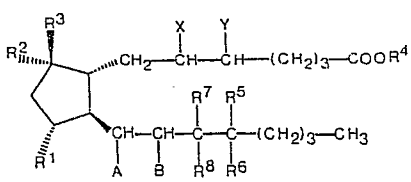

worin $R^1$ Wasserstoff oder Niederalkyl; $R^2$ Hydroxy; $R^3$ Wasserstoff; oder $R^2$ und $R^3$ zusammen Oxo; $R^4$ Wasserstoff oder Niederalkyl; $R^5$ Fluor, Niederalkyl oder Trifluormethyl; $R^6$ Wasserstoff, Fluor oder Niederalkyl; $R^7$ Wasserstoff; $R^8$ Hydroxy; oder $R^7$ und $R^8$ zusammen Oxo; A und B, und X und Y, für sich allein Wasserstoff oder zusammen eine Kohlenstoff- Kohlenstoffbindung bedeuten; mit der Bedingung, dass $R^2$ und $R^3$ zusammen Oxo darstellen, wenn $R^7$ und $R^8$ zusammen Oxo sind; und mit der weiteren Bedingung, dass A und B Wasserstoff darstellen, wenn $R^7$ Wasserstoff ist; und mit der dritten Bedingung, dass X und Y eine Kohlenstoff- Kohlenstoffbindung darstellen, wenn $R^1$ und $R^7$ gleichzeitig Wasserstoff sind; deren Enantiomere und Racemate weisen therapeutische, z.B. blutdrucksenkende und anti-ulcerogene Wirkungen auf.

Die Erfindung betrifft solche Prostaglandinderivate, deren Herstellung und pharmazeutische Präparate enthaltend solche Prostaglandinderivate, sowie neue Zwischenprodukte für die Herstellung dieser Prostaglandinderivate.

EP 0 000 207 A1

4. Juli 1978
0000207

RAN 4303/7

F. Hoffmann-La Roche & Co. Aktiengesellschaft, Basel/Schweiz

Neue Prostaglandinderivate, deren Herstellung und pharmazeutische Präparate und Zwischenprodukte.

Die vorliegende Erfindung betrifft neue Prostaglandinderivate der Formel I

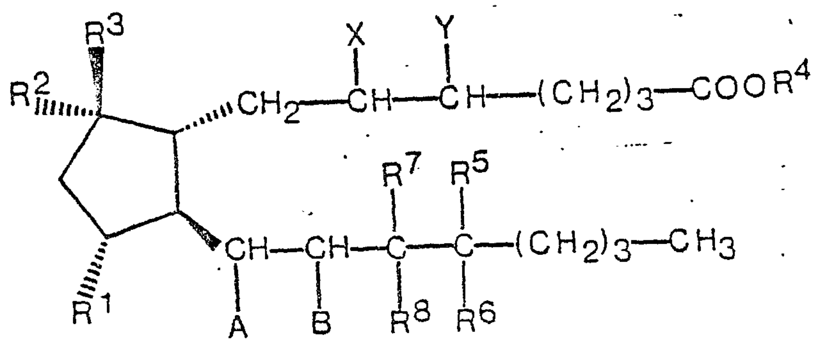

I

worin $R^1$ Wasserstoff oder Niederalkyl; $R^2$ Hydroxy; $R^3$ Wasserstoff; oder $R^2$ und $R^3$ zusammen Oxo; $R^4$ Wasserstoff oder Niederalkyl; $R^5$ Fluor, Niederalkyl oder Trifluormethyl; $R^6$ Wasserstoff, Fluor oder Niederalkyl; $R^7$

Wasserstoff; $R^8$ Hydroxy; oder $R^7$ und $R^8$ zusammen Oxo; A und B, und X und Y, für sich allein Wasserstoff oder zusammen eine Kohlenstoff-Kohlenstoffbindung bedeuten; mit der Bedingung, dass $R^2$ und $R^3$ zusammen Oxo darstellen, wenn $R^7$ und $R^8$ zusammen Oxo sind; und mit der weiteren Bedingung, dass A und B Wasserstoff darstellen,wenn $R^7$ Wasserstoff ist; und mit der dritten Bedingung, dass X und Y eine Kohlenstoff- Kohlenstoffbindung darstellen, wenn $R^1$ und $R^7$ gleichzeitig Wasserstoff sind;

deren Enantiomere und Racemate.

Die Erfindung betrifft weiterhin ein Verfahren zur Herstellung von Prostaglandinderivaten der Formel I und neue Zwischenprodukte, die in diesem Prozess auftreten. Weiterhin betrifft die Erfindung neue pharmazeutische Präparate,die Prostaglandinderivate der Formel I enthalten.

Der hier verwendete Ausdruck "Niederalkyl" bezieht sich auf geradkettige oder verzweigte Alkylgruppen mit 1 bis 7 C-Atomen wie Methyl und Aethyl. Der Ausdruck "Niederalkancarbonsäure" bezieht sich auf Alkancarbonsäure mit 1-7 C-Atomen wie Ameisensäure und Essigsäure. Der Ausdruck Halogen oder Halo bezieht sich auf Fluor, Chlor, Brom und Jod.

Erfindungsgemäss können alle Verbindungen mit einem oder mehreren asymmetrischen Kohlenstoffatomen als Racemate hergestellt werden. Diese Racemate können an geeigneter Stelle bei der Herstellung nach an sich bekannten Methoden gespalten werden, wobei dann als Folgeprodukte die entsprechenden, optisch reinen Enantiomeren erhalten werden. Andererseits können die optisch aktiven Enantiomeren der Formel I aus optisch aktiven Verbindungen der Formel II, hergestellt werden.

In den Strukturformeln bedeutet eine keilförmige Linie (▼) einen Substituenten der sich in Beta-Stellung (oberhalb der Molekülebene) befindet, eine unterbrochene Linie (/////) bezeichnet einen Substituenten in Alpha-Stellung (unterhalb der Molekülebene). Die Darstellung einer bestimmten Strukturformel wurde aus Gründen der Einfachheit gewählt und ist so zu verstehen, dass sie auch Enantiomere und Racemate einschliesst.

Der Ausdruck "Aryl" bezeichnet sowohl einkernige aromatische Kohlenwasserstoffgruppen wie Phenyl oder Tolyl die unsubstituiert oder auch in einer oder mehreren Stellungen mit Alkylendioxy, Halogen, Nitro, oder Niederalkoxy substituiert sein können, als auch mehrkernige Arylgruppen wie Naphthyl, Anthryl, Phenanthryl und Azulyl, die mit einer oder mehreren der vorgenannten Gruppen substituiert sein können. Bevorzugte Arylgruppen sind substituierte und unsubstituierte einkernige Arylgruppen, insbesondere Phenyl. Der Ausdruck Aryl, Niederalkyl bezeichnet Gruppen worin Aryl und Niederalkyl wie oben definiert sind, insbesondere Benzyl.

Der Ausdruck "hydrolysierbare Ester oder Aethergruppe" bezeichnet eine beliebige Ester- oder Aethergruppe, die durch Hydrolyse in die Hydroxygruppe übergeführt werden kann. Beispiel für solche Estergruppen sind die, in denen der Acylrest sich von einer Niederalkansäure, einer

Arylniederalkansäure, Phosphorsäure, Kohlensäure oder Niederalkandisäure ableitet. Solche Estergruppen können aus Säureanhydriden und Säurehalogeniden, insbesondere Chloriden oder Bromiden, gebildet werden, wobei die Niederalkancarbonsäureanhydride, zum Beispiel Acetanhydrid und Capronsäureanhydrid; Aryl-niederalkansäureanhydride zum Beispiel Benzosäureanhydrid, Niederalkandisäureanhydride, zum Beispiel Bernsteinsäureanhydrid, und Chlorameisensäureester, zum Beispiel Trichloraethylchlorameisensäureester bevorzugt sind. Eine besonders geeignete Aetherschutzgruppe ist beispielsweise der Tetrahydropyranylaether oder der 4-Methoxy-5,6-dihydro-2H-pyranylaether. Andere solche Aether sind Arylmethylaether, wie Benzyl, Benzhydryl oder Tritylläther, oder α-Niederalkoxyniederalkyläther, zum Beispiel Methoxymethyl oder Allyläther oder Trialkylsilyläther wie Trimethylsilylaether oder Dimethyl-tert.-silyläther.

Eine Gruppe von Verbindungen der Formel I kann durch die Formel IA

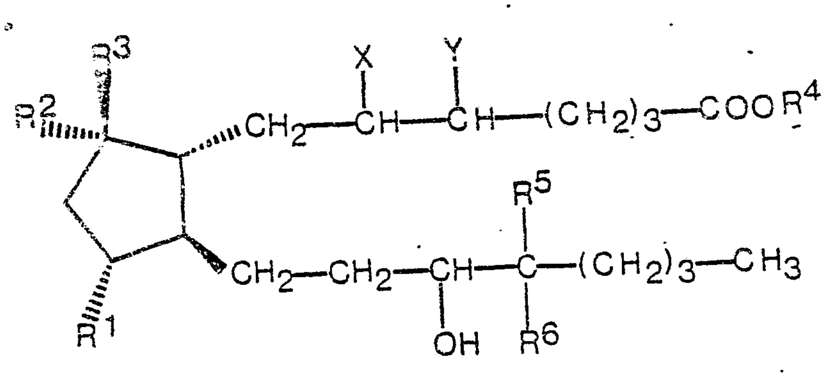

IA

worin $R^1$ bis $R^6$, X und Y die obige Bedeutung haben, mit der Bedingung, dass X und Y eine Kohlenstoff- Kohlenstoffbindung darstellen, wenn $R^1$ Wasserstoff ist,

dargestellt werden.

Eine andere, besonders interessierende Gruppe

BAD ORIGINAL

von Verbindungen der Formel I hat die Formel IB

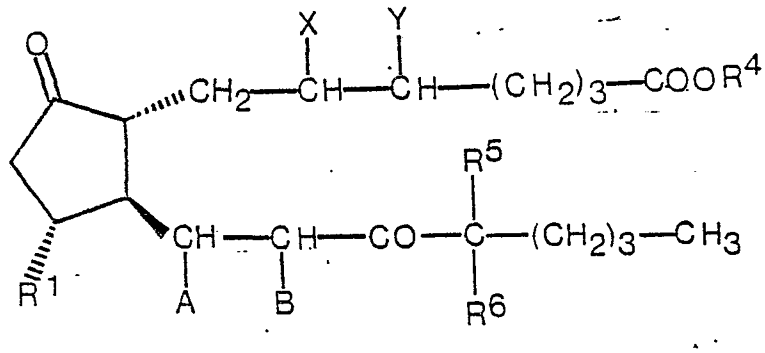

IB

worin $R^1$, $R^4$, $R^5$, $R^6$, A, B, X und Y die obige Bedeutung haben.

Die Verbindungen der Formeln I werden erfindungsgemäss dadurch hergestellt, dass man

a) eine Verbindung der Formel II

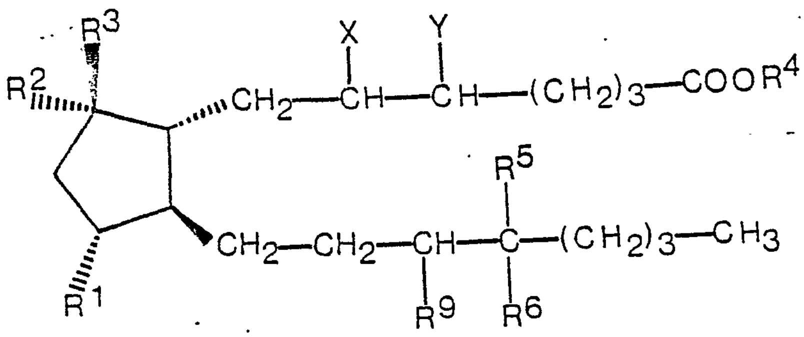

II

worin $R^9$ eine durch eine hydrolysierbare Aether- oder Estergruppe geschützte Hydroxygruppe darstellt und $R^1$ bis $R^6$, X und Y die obige Bedeutung haben

oder ein Enantiomer oder Racemat davon der Hydrolyse der Schutzgruppe $R^9$ unterwirft und gewünschtenfalls eine durch X und Y dargestellte Kohlenstoff- Kohlenstoffbindung hydriert, und/oder eine Hydroxygruppe zur Oxogruppe oxidiert, oder

b) die Hydroxygruppe(n) in einer Verbindung der Formel III

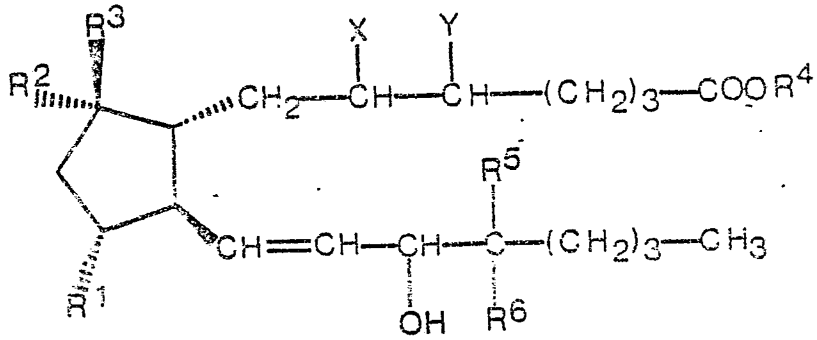

III

worin $R^1$ bis $R^6$, X und Y die obige Bedeutung haben,

oder eines Enantiomers oder Racemats davon zu einer Verbindung der Formel I, worin $R^2$ und $R^3$ bzw. $R^7$ und $R^8$ Oxo darstellen, oxidiert.

Die Hydrolyse der geschützten Hydroxygruppe $R^9$ kann durch konventionelle Aether- und Esterhydrolysemethoden durchgeführt werden.

Saure wässrige Hydrolyse kann verwendet werden, wenn die Hydroxygruppe durch eine Aetherbindung geschützt ist. Zu den bevorzugten Methoden der Aetherhydrolyse gehört die Behandlung einer Verbindung der Formel II mit einer wässrigen anorganischen Säure. Andererseits kann, wenn $R^9$ eine Estergruppe enthält, die Hydroxygruppe durch Behandlung mit einer Base in wässrigem Medium regeneriert werden. Jede konventionelle Methode der Esterhydrolyse kann bei dieser Umwandlung verwendet werden. Unter den bevorzugten Basen ist wässriges Natriumhydroxid zu nennen. Basische Hydrolyse führt auch zu einer Spaltung der Estergruppe in einer Verbindung der Formel II, in der $R^4$ Niederalkyl ist, wobei man eine Verbindung der Formel I erhält, in der $R^4$ Wasserstoff ist. Wenn eine Verbindung der Formel I mit $R^4$ = Alkyl hergestellt werden soll, sollte eine

BAD ORIGINAL

Aetherschutzgruppe $R^9$, wie Tetrahydropyranyloxy verwendet werden.

Die Hydrierung einer durch X und Y dargestellten C-C-Bindung im Anschluss an die Hydrolyse von $R^9$ in einer Verbindung der Formel II kann durch jede konventionelle Hydriermethode,wie katalytische Hydrierung,bewerkstelligt werden.

Zu den bevorzugten Hydriermethoden gehört die Hydrierung in Gegenwart eines Katalysators wie Platin oder Platinoxyden. Jeder konventionelle Hydrierkatalysator kann hierbei Verwendung finden. Die Reaktionsbedingungen sind gleichfalls die für gewöhnliche Hydrierreaktionen.

Die Oxidation von Hydroxygruppen im Anschluss an die Hydrolyse von $R^9$ in einer Verbindung der Formel II und die Oxidation von Hydroxygruppen in einer Verbindung der Formel III können durch konventionlle Oxidationsmittel durchgeführt werden,die eine Hydroxygruppe zur Ketogruppe oxidieren. Bevorzugte Oxidationsmittel sind Chromat-Oxidationsmittel wie Chromtrioxid. Als Reaktionsbedingungen kommen die für diese Oxidationsreaktionen üblichen Bedingungen in Betracht.

Je nachdem,welche besondere Form der Verbindung der Formel I gewünscht wird, wird als Ausgangsmaterial eine Verbindung II oder III verwendet die entweder ein Racemat ist oder in optisch aktiver Form vorliegt.

Die Ausgangsverbindung der Formel II und III können wie nachstehend beschrieben hergestellt werden.

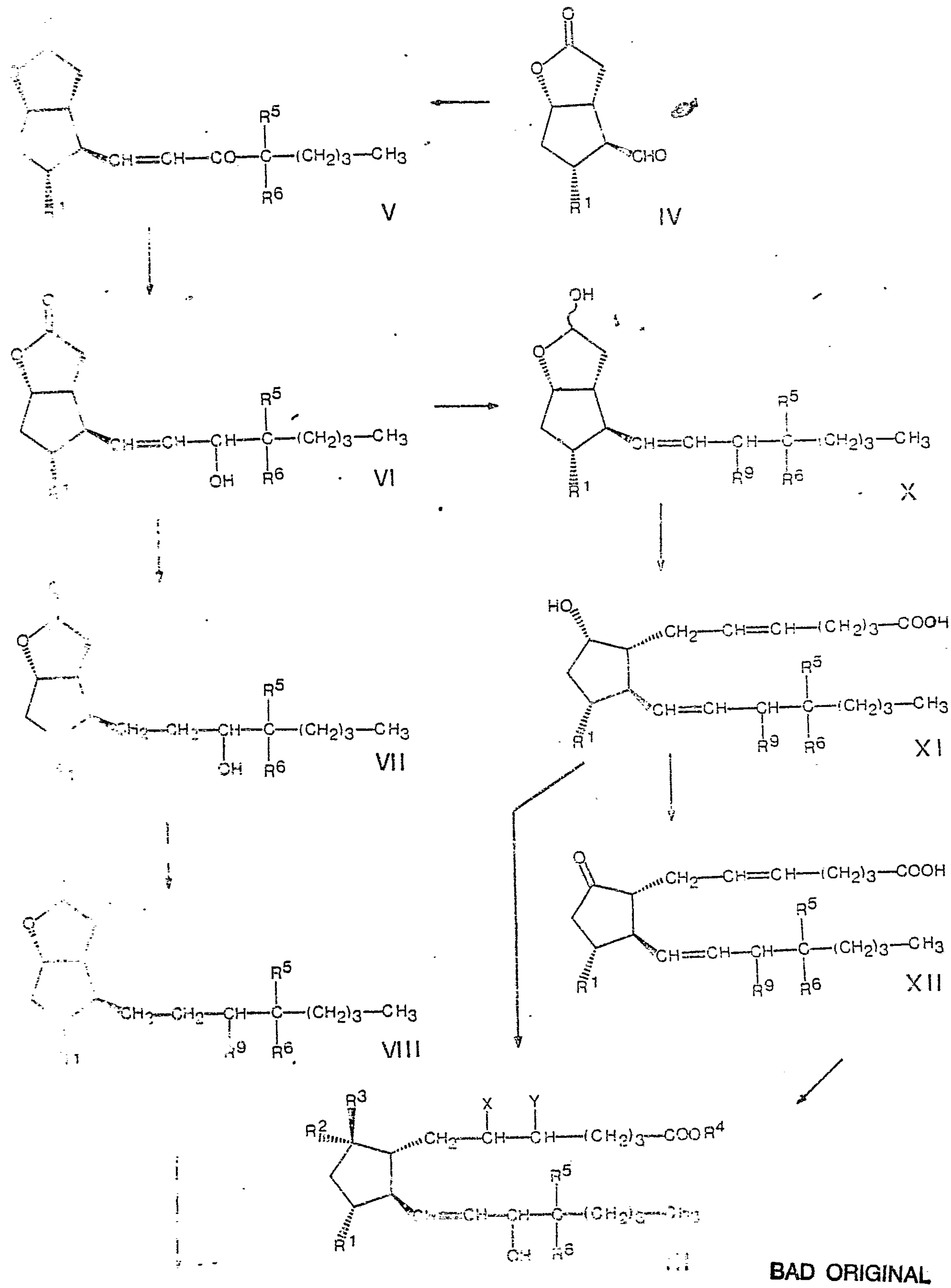

V IV

VI X

VII XI

VIII XII

I

BAD ORIGINAL

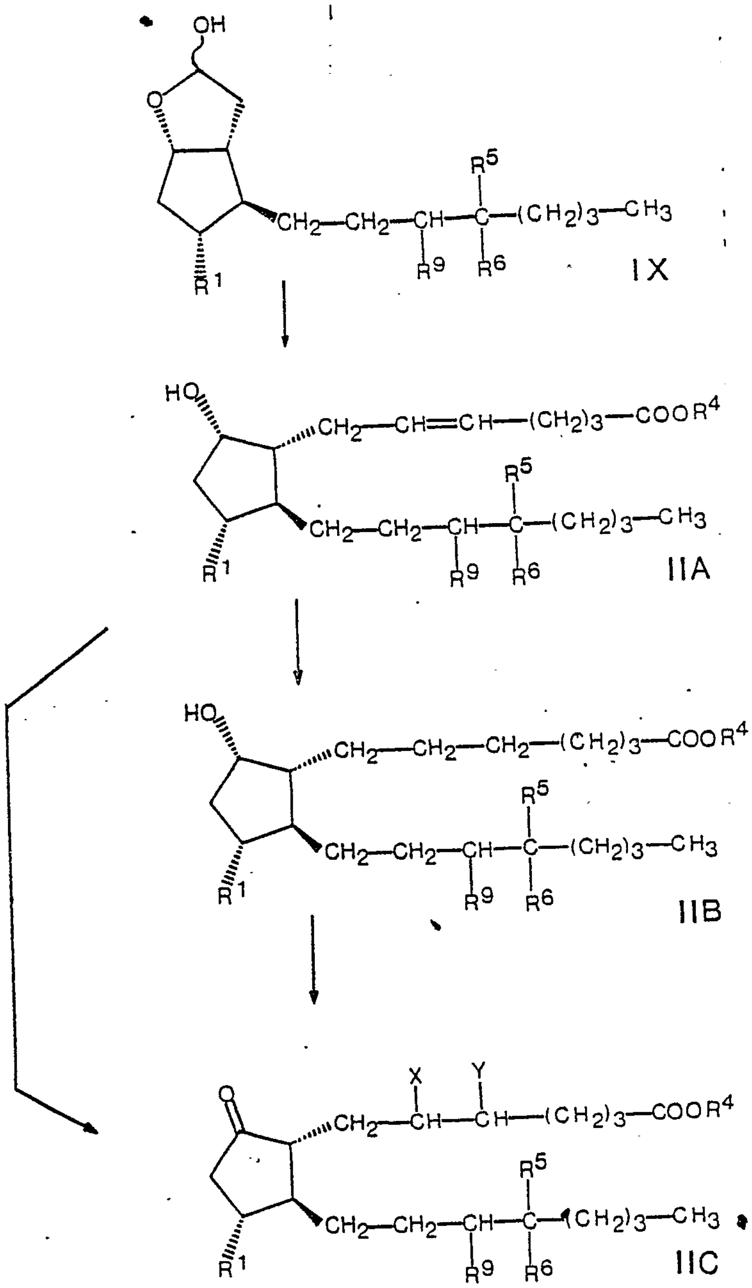

IX

IIA

IIB

IIC

0000207

Die Verbindung der Formel IV kann in die Verbindung der Formel V durch Umsetzung mit einem Phosphoniumsalz der Formel XIII

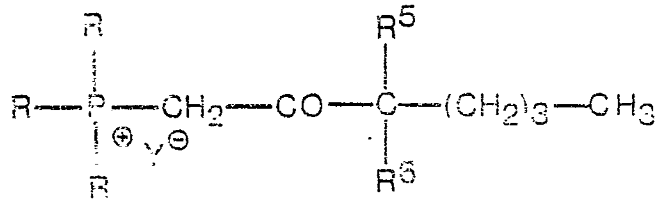

XIII

worin $R^5$ und $R^6$ die obige Bedeutung haben, und $R^6$ Aryl oder Di(Niederalkylamino) und Y Halogen darstellt,

oder einem Phosphonat der Formel XIV

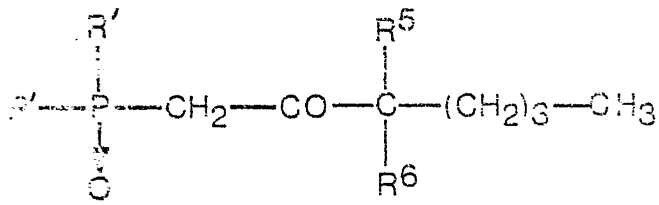

XIV

worin $R^5$ und $R^6$ die obige Bedeutung haben, und R' Aryl, Aryloxy oder Niederalkoxy darstellen,

umgewandelt.

Die Umsetzung einer Verbindung der Formel IV mit dem Phosphoniumsalz zu einer Verbindung der Formel V ist eine Wittig-Reaktion. Bei dieser Reaktion können die für Wittig-Reaktionen konventionellen Reaktionsbedingungen angewendet werden.

Die Umsetzung einer Verbindung der Formel IV mit dem Phosphonat zu einer Verbindung der Formel V ist eine Horner-Reaktion, für diese Reaktion kommen die für Horner-Reaktion üblichen Reaktionsbedingungen in Betracht.

Die Verbindung der Formel VI kann durch Umsetzung

BAD ORIGINAL

einer Verbindung der Formel V mit einem Reduktionsmittel erhalten werden. Für die Durchführung dieser Reaktionen können alle konventionellen Reduktionsmittel verwendet werden, die selektiv eine Ketogruppe zur Hydroxygruppe reduzieren. Bevorzugte Reduktionsmittel sind Hydride, insbesondere Aluminiumhydride wie Alkalimetallaluminiumhydride und Borhydride wie Alkalimetallborhydride, wobei Zinkborhydrid besonders bevorzugt ist. Bei der Durchführung dieser Reaktion sind Temperatur und Druck nicht kritisch, die Reaktion kann bei Raumtemperatur und Atmosphärendruck oder bei erhöhten oder verminderten Temperaturen und Drucken durchgeführt werden. Im allgemeinen ist es vorzuziehen, die Reaktion bei einer Temperatur von -30°C bis zur Rückflusstemperatur des Reaktionsgemisches durchzuführen. Die Reduktion kann in Gegenwart eines inerten organischen Lösungsmittels durchgeführt werden. Hierbei kann jedes konventionelle inerte organische Lösungsmittel oder Wasser verwendet werden, zum Beispiel die oben erwähnten inerten organischen Lösungsmittel. Bevorzugt sind als Lösungsmittel Methanol, Dimethoxyäthylenglykose und Aether wie Tetrahydrofuran, Diäthyläther und Dioxan.

Die Umwandlung einer Verbindung der Formel VI in eine Verbindung der Formel VII wird durch Hydrierung durchgeführt. Die Hydrierung kann unter den Bedingungen die oben für die Hydrierung einer C=C-Bindung im Anschluss an die Hydrolyse von $R^9$ in einer Verbindung der Formel II beschrieben sind, durchgeführt werden.

Die Verbindung der Formel VII wird in eine Verbindung der Formel VIII durch Veresterung oder Verätherung der freien Hydroxygruppe mit einer hydrolysierbaren Aether- oder Esterschutzgruppe übergeführt. Diese Veresterung oder Verätherung kann nach den üblichen Veresterungs- und Verätherungsverfahren durchgeführt werden. Bevorzugte Estergruppen sind Niederalkanoyloxy, insbesondere Acetoxy, eine bevorzugte Aethergruppe ist Tetrahydropyranyloxy.

Die Verbindung der Formel VIII wird durch Behandlung mit einem Reduktionsmittel in eine Verbindung der Formel IX

0000207

übergeführt. Hierbei kann jedes konventionelle Reduktionsmittel verwendet werden das ein Lacton reduktiv zum Lactol reduziert. Zu diesen Reduktionsmitteln gehören Hydride, wie Alkalimetallborhydride, insbesondere Di-isobutylaluminiumhydrid. Die Reaktion kann auch mittels Di(verzweigtkettig-niederalkyl)-boranen wie Bis-(3-methyl-3-butyl)-boranen durchgeführt werden. Bei dieser Reaktion sind Temperatur und Druck nicht von kritischer Bedeutung, die Reaktion kann bei Raumtemperatur und Atmosphärendruck oder erhöhten oder verminderten Temperaturen und Drucken ausgeführt werden. Im allgemeinen ist es vorzuziehen, die Reaktion bei Temperaturen zwischen -70°C und Raumtemperatur (20°C) auszuführen. Die Reduktion kann in Gegenwart eines inerten organischen Lösungsmittels ausgeführt werden, wofür sich jedes konventionelle inerte organische Lösungsmittel eignet. Unter den bevorzugten Lösungsmitteln sind Dimethoxyäthylenglykol und Aether wie Tetrahydrofuran, Diäthyläther und Dioxan zu nennen.

Verbindung der Formel II-B in der $R^4$ Wasserstoff ist, werden aus Verbindung der Formel IX durch Umsetzung mit einem Phosphoniumsalz der Formel XV

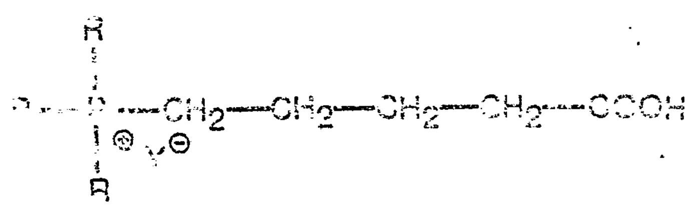

XV

worin R Aryl oder Di(niederalkyl)Amino und Y Halogen darstellen

hergestellt.

Die Verbindung der Formel IX reagieren mit dem Phosphoniumsalz unter Bildung einer Verbindung der Formel II-B mit überwiegender cis-Konfiguration der Doppelbindung in 5-Stellung der Seitenkette in einem Lösungsmittel, das Hexamethylphosphoramid enthält und unter Verwendung

BAD ORIGINAL

von Natrium-bis-trimethylsilylamid als Base. Wenn andere Lösungsmittel als Hexamethylphosphoramid und andere Basen als Natrium-bis-trimethylsilylamid verwendet werden, erhält man die Verbindung der Formel II-B in schlechterer Ausbeute. Es können aber auch konventionelle inerte organische Lösungsmittel allein oder im Gemisch mit Hexamethylphosphoramid verwendet werden. Andererseits kann das Lösungsmittelsystem allein aus Hexamethylphosphoramid bestehen. Bei der Durchführung dieser Reaktion sind Temperatur und Druck nicht von kritischer Bedeutung, die Reaktion kann bei Raumtemperatur und Normaldruck oder bei höheren oder niederen Temperaturen durchgeführt werden. Im allgemeinen ist es zweckmässig, bei Temperaturen von 0-50°C zu arbeiten.

Verbindungen der Formel II-A, in denen $R^4$ Wasserstoff ist, können gewünschtenfalls in entsprechende Verbindungen, in denen $R^4$ Niederalkyl ist durch konventionelle Veresterungsverfahren, zum Beispiel durch Reaktion mit Diazomethan, umgewandelt werden.

Die Umwandlung von Verbindung der Formel VI in Verbindung der Formel X kann in Analogie zu Umwandlung von Verbindung der Formel VIII in Verbindung der Formel IX durchgeführt werden. In ähnlicher Weise kann die Umwandlung X→XI in Analogie zu IX→II-A durchgeführt werden und die Hydrierung der Doppelbindung in der Umwandlung von XI oder XII zu III kann, wie für die Reaktion II-A→II-B beschrieben, durchgeführt werden. Die Veresterung der Carboxygruppe in Verbindung mit der Verbindung XI und XII kann in Analogie zur Veresterung einer Carboxygruppe in der Verbindung II-A durchgeführt werden.

Die Verbindungen der Formel I, deren optische Antipoden und Racemate sind als Pharmazeutika von Wert. Insbesondere sind sie wertvoll als Bronchodilatoren, anti-

BAD ORIGINAL

0000207

sekretorische Mittel, [illegible] Blutdrucksenker und zur Bekämpfung von gastrischer Hyperacidität.

Die Verbindungen der Formel I-A, deren optische Antipoden und Racemate sind wirksam als antisekretorische, blutdrucksenkende, und antiulcerogene Mittel. Die Verbindungen der Formel I-B und deren optischen Antipoden und Racemate in denen A und B eine Doppelbindung bilden, sind ebenfalls wirksam als [illegible] Mittel, Blutdrucksenker und antiulcerogene Mittel.

Die Verbindung der Formel I-B, deren optische Antipoden und Racemate, in denen A und B Wasserstoff sind, sind antisekretorisch und antiulcerogen wirksam.

Die Wirksamkeit der erfindungsgemässen Verbindungen als Blutdrucksenker zeigt sich bei der Verabreichung [illegible] 17-methyl-16R-fluor-9,15-dioxoprosta-(Z)-5-(E)-[illegible]säure an Ratten in dem folgenden Test:

Für den Versuch dienten männliche Charles River-[illegible] im Gewicht von 170-210 Gramm. Durch unilaterale [illegible] und anschliessende subcutane Implantationen eines [illegible] mg-Pellets Dioxycortisonacetat wurde ein DOCA-Hochdruck induziert. Die Tiere wurden in Einzelkäfige gesetzt und erhielten eine 0,9 gewichtsprozentige wässrige Natriumchloridlösung und Futter ad libitum. [illegible] dem Eingriff und der Entwicklung des Hochdrucks, (systolischen Blutdruck von über 150 mmHg) lässt man [illegible] Wochen verstreichen. Der systolische Blutdruck wird indirekt am Schwanz der nicht anästhetisierten Tiere (5-10 [illegible] 37-[illegible]°C in Halterungen fixiert) mittels [illegible] (photoelektrischer [illegible] Wandler und die [illegible] verbunden. [illegible] werden vor der [illegible] 1, 3, 5, 24, [illegible] Stunden nach der [illegible] Verbin-

BAD ORIGINAL

dungen vorgenommen. Die zu verabreichenden Verbindungen werden in einem Gemisch von Tris(hydroxymethyl)aminomethan in 95% Aethanol (5% Gew./Vol.) oral verabreicht. Als Placebo wurde ein Gemisch von Tris(hydroxymethyl)aminomethan in Aethanol verwendet.

Die Versuchsresultate sind in der nachstehenden Tabelle angegeben:

0000207

Wirkung auf den systolischen Blutdruck
bei DOCA-Na Hochdruck bei Ratten bei oraler Applikation

| Dosis (mg/kg p.o) | N | Systolischer Blutdruck Zeit (Stunden) 0 | 1 | 3 | 6 | 24 | 48 | 72 |
|---|---|---|---|---|---|---|---|---|
| 1.0 | 6 | 196±6 | 200±6 | 205±6 | 203±8 | 199±6 | | – |
| 5.0 | 6 | 197±3 | 175±2* | 167±4* | 166±3* | 163±4* | 171±7* | 193±6 |

$p < .05$

BAD ORIGINAL

Die antisekretorische und antiulcerogene Aktivität wurde in folgender Versuchsanordnung ermittelt:
Verbindung A = Nat.11R-methyl-16R-fluor-15R-hydroxy-9-oxo-prost-(Z)-5-en-säure
Verbindung B = Nat.11R-methyl-16R-fluor-9,15-dioxo-prost-(Z)-5-en-säure.

Die Verbindungen wurden an nicht anästhesierten Ratten mit akuter gastrischer Fistel getestet. Am Tag vor dem Experiment wurden gefastete weibliche Ratten (mittleres Gewicht 250 g) chirurgisch katheterisiert in der vena cava inferior (zwecks konstanter Infusion von Kochsalzlösung und Verabreichung der Testverbindungen); am Gallengang (um Galle und Pancreassekretion abzuleiten, die durch Rückfluss den Mageninhalt verunreinigen können); am Mageneingang (zwecks Infusion eines kleinen Volumens Wasser während des Experiments); und am Magenausgang (zum Sammeln des Mageninhaltes und zur kontinuierlichen Ueberwachung des pH durch eine Mikroelektrode). Am Tag des Experimentes wurde vor der Verabreichung der Verbindungen der Magen während 60 Minuten mit Wasser gespült. Während dieser Basisperiode betrug das pH (gemessen in 10-Minuten-Intervallen) des Sekretionsflusses bei jedem Tier etwa 1,5. Nach der Basisperiode wurde die zur verabreichende Verbindung, in Kochsalzlösung gelöst in einer Dosis von 16 µg/kg intravenös verabreicht und es wurden für die Dauer von 60 Minuten Proben kontinuierlich gesammelt. Gemessen wurde das pH, das Volumen, die Gesamt-Säure (µAeq/ml) und die gesamte Säureausschüttung während 10 Minuten (µAeq/10 Minuten). Die Resultate sind in der nachstehenden Tabelle zusammengefasst.

Antisekretorische Wirksamkeit bei der wachen Ratte mit akuter gastrischer Fistel

| Minuten nach Verabreichung | Verbindung A[e] |  |  |  | Verbindung B[e] |  |  |  |
|---|---|---|---|---|---|---|---|---|
|  | pH[a] | Volumen[b] | Säure Konz.[c] | Säure Ausschüttung[d] | pH[a] | Volumen[b] | Säure Konz.[c] | Säure Ausschüttung[d] |
| 10 | 5 | 0 | 0 | 4 | 2 | 9 | 33 | [illegible] |
| 20 | 6 | 0 | 22 | 17 | 2 | 30 | 6 | [illegible] |
| 30 | 4 | 58 | 20 | 33 | 2 | 4 | 11 | 15 |
| 40 | 5 | 21 | 23 | 26 | 2 | 11 | 2 | 14 |
| 50 | 9 | 9 | 38 | 43 | 2 | 16 | 15 | 20 |
| 60 | 9 | 0 | 22 | 25 | 5 | 35 | 19 | 48 |
| N[f] = | 3 |  |  |  | [illegible] |  |  |  |

a % Zunahme über Basisperiode.

b % Hemmung.

c % Hemmung (berechnet aus µAeq/ml der Gesamtsäurekonzentration

d % Hemmung (berechnet aus µAeq/10 Minutenperiode des gesamten Säureausstosses

e Abgerundete Werte; 0 = kein Effekt oder Zunahme der Wasserstoffionenkonzentrationen

f Anzahl der Versuchstiere.

BAD ORIGINAL

Die Verbindungen der Formel I können in pharmazeutischen und veterinärmedizinischen Präparaten Verwendung finden. Die Präparate können als Tabletten, Pillen, Pulver, Kapseln, Injektionslösungen, Lösungen, Suppositorien, Emulsionen, Dispersionen, als Futterpremix und in anderer geeigneter Form vorliegen. Die Präparate enthalten die Verbindungen der Formel I zweckmässig im Gemisch mit einem nicht-toxischen pharmazeutischen organischen anorganischen Träger. Typische pharmazeutisch anwendbare Träger sind beispielsweise Wasser, [illegible], [illegible], [illegible] Magnesiumstearat, Talk, vegetabile Oele, Polyalkylenglycole, Vaseline und andere üblicherweise verwendete pharmazeutische Träger. Die Präparate können auch Hilfsstoffe wie Emulgatoren, Konservierungs- und Netzmittel enthalten, beispielsweise Sorbitan-monolaurat, Triäthanolaminoleat, Polyoxyäthylensorbitan und Dioctylnatriumsulfosuccinat.

Die zu verabreichende Dosierung der Verbindungen variiert mit der Art der Verbindung, der Art und Stelle der Verabreichung. Eine typische Verabreichungsmethode für die Verbindung der Formel I ist die Injektion. Hierbei kann eine sterile Lösung, die die Verbindung der Formel I enthält, intravenös in einer Dosis von 0,1 Mikrogramm bis 0,3 Mikrogramm pro Tag und kg Körpergewicht verabreicht werden. Die so zu verabreichenden Verbindungen können zum Beispiel als sterile wässrige Lösung vorliegen, die ein Mittel enthalten, welches die Absorption verzögert, wie Aluminiummonostearat.

Zur Verabreichung der Verbindung der Formel I an Haus- oder Laboratoriumstiere können die Verbindungen in Form eines Futterpremix, zum Beispiel im Gemisch mit getrockneten Fischmehl oder Hafermehl, vorliegen. Das Premix wird dann dem eigentlichen Futter beigemischt.

Die nachfolgenden Beispiele erläutern die Erfindung. In den Beispielen sind die Temperaturen in Celsiusgraden angegeben, die Bezeichnung Aether bezieht sich auf

BAD ORIGINAL

Diäthyläther. Das verwendete Jones-Reagens ist eine [illegible] molare Lösung von Chromtrioxid in Schwefelsäure. [illegible] bedeutet Hexamethylphosphoramid.

ORIGINAL

## Beispiel 1

Zu einer Lösung von 100 mg nat-11R-Methyl-16R-fluor-15R-hydroxy-9-oxoprosta-5-(Z).13(E)-diensäure oder nat-11R-Methyl-16R-fluor-15S-hydroxy-9-oxoprosta-5(Z).13(E)-diensäure in 10 ml einer Diäthyläther-Aceton-Lösung (5:[illegible] Volumteile) wurden 0.1 ml Jones-Reagenz gegeben. Nach 10 Minuten wurde das Reaktionsgemisch mit 50 ml Aether versetzt, die Lösung mit verdünnter wässriger [illegible]lösung gewaschen, die organische Phase über Magnesiumsulfat getrocknet und das Lösungsmittel unter vermindertem Druck entfernt. Das Rohprodukt wurde durch Chromatographie an Silicagel gereinigt und lieferte 80 mg nat 11R-Methyl-16R-fluor-9,15-dioxoprosta-5(Z),13(E)-diensäure als farblose Flüssigkeit, $[\alpha]_D^{25}$ -13°C (in Aethanol).

## Beispiel 2

In Analogie zu Beispiel 1 wurde aus nat-11R-Methyl-16S-fluor-15R-hydroxy-9-oxoprosta-5(Z),13(E)-diensäure die nat-11R-Methyl-16S-fluor-9,15-dioxoprosta-5(Z),13(E)-diensäure erhalten.

## Beispiel 3

In Analogie zu Beispiel 1 wurde aus nat-16R-Fluor-15R-hydroxy-9-oxoprosta-5(Z),13(E)-diensäure die nat-16R-Fluor-9,15-dioxoprosta-5(Z),13(E)-diensäure als farbloses Oel erhalten.
Massenspektrum: $M^+$ (m/e 352);
UV (Aethanol) $\lambda$ max 238 nm ($\varepsilon$ = 10600).

## Beispiel 4

In Analogie zu Beispiel 1 wurde aus nat-16R-Trifluor-methyl-15R-hydroxy-9-oxoprosta-5(Z),13(E)-diensäure die nat-16R-Trifluormethyl-9,15-dioxoprosta-5(Z),13(E)-diensäure erhalten.

BAD ORIGINAL

## Beispiel 5

In Analogie zu Beispiel 1 wurde aus nat-11R,16,16-Trimethyl-15R-hydroxy-9-oxoprosta-5(Z),13(E)-diensäure die nat-11R-16,16-Trimethyl-9,15-dioxoprosta-5(Z),13(E)-diensäure als farbloses Oel erhalten.
Massenspektrum: $M^+$ (m/e 376)
UV (Aethanol) $\lambda$ max 235 nm ($\varepsilon$ = 7425)

## Beispiel 6

750 mg nat - 11R-Methyl-116R-fluor-15R-[2-tetrahydro-(2H)-pyranyloxy]-9-oxoprost-5(Z)-ensäure wurde in 15 ml eines 5:1 Gemisches von Essigsäure und Wasser 1.5 Stunden auf 49° erwärmt. Das Lösungsmittel wurde dann unter Hochvakuum entfernt und der Rückstand chromatographiert. Man erhielt 400 mg nat-11R-Methyl-16R-fluor-15R-hydroxy-9-oxoprost-5(Z)-ensäure als dickes farbloses Oel.
Massenspektrum: m/4 370, 352, 332.

Das Ausgangsmaterial wurde wie folgt hergestellt:

Ein Gemisch von 1 g 3,3aR,4,5,6,6aS-Hexahydro-4R-[4R-fluor-3R-hydroxy-1(E)-octenyl]-5R-methyl-2H-cyclopenta-[b]furan-2-on, 100 mg vorreduziertes $PtO_2$ und 75 ml Aethylacetat wurde bei Raumtemperatur und Atmosphärendruck bis zur theoretischen Wasserstoffaufnahme hydriert. Das Gemisch wurde dann filtriert und das Lösungsmittel unter vermindertem Druck entfernt. Verreiben des Rückstandes mit Hexan lieferte 700 mg 3,3aR,4,5,6,6aS-Hexahydro-4R-(4R-fluor-3R-hydroxy-octanyl)-5R-methyl-2H-cyclopenta[b]furan-2-on, Schmelzpunkt 52-55°C.

Zu einer Lösung von 730 mg 3,3aR,4,5,6,6aS-Hexahydro-4R-(4R-fluor-3R-hydroxy-octanyl)-5R-methyl-2H-cyclopenta-[b]furan-2-on in 50 ml Methylenchlorid wurden 50 mg Paratoluolsulfonsäure und 1 ml Dihydropyran gegeben. Die Lösung wurde bei Raumtemperatur 3 Stunden gerührt, danach mit 10

Tropfen gesättigter Natriumbicarbonat... und ... Aether versetzt. Die organische Schicht wurde mit gesättigter wässriger Natriumchloridlösung gewaschen, über Magnesiumsulfat getrocknet und unter vermindertem Druck eingeengt. Reinigung des Rückstandes durch Chromatographie lieferte 3,3aR,4,5,6,6aS-Hexahydro-4R-(4R-fluor-3R-[2-tetrahydro-(2H)-pyranyloxy]-octanyl)-5R-methyl-2H-cyclopenta[b]furan-2-on. Dieses Rohprodukt wurde in 10 ml trockenem ... gelöst und unter Argon bei -70° mit 2 ml einer ... Lösung von Diisobutylaluminiumhydrid versetzt. Nach einer Stunde wurde langsam Methanol zugegeben und das Reaktionsgemisch wurde auf Raumtemperatur aufgewärmt und schliesslich mit 2 ml Wasser versetzt. Das Gemisch wurde über ein Filter-Hilfsmittel filtriert und das Lösungsmittel unter vermindertem Druck entfernt und man erhielt nach Chromatographie 3,3aR,4,5,6,6aS-Hexahydro-4R-(4R-fluor-3R-[2-tetrahydro-(2H)-pyranyloxy]-ocatanyl)-5R-methyl-2H-cyclopenta[b]furan-2-ol als dickes farbloses Oel.

Massenspektrum: m/e 372, 354, 269.

... 4.8 g Natrium-bis-(trimethylsilyl)-amid wurden in 50 ml HMPA gelöst, wobei Argon kontinuierlich durch die Lösung geleitet wurde. Danach wurden 5.8 g (4-Carboxybutyl)-triphenylphosponiumbromid zugesetzt und die Aufschlämmung wurde bis zur Bildung einer tiefroten Lösung gerührt. Zu dieser Lösung wurden dann 2 g 3,3aR,4,5,-6,6aR-Hexahydro-4R-(4R-fluor-3R-[tetrahydro-(2H)-2-pyranyloxy]-octanyl)-5R-methyl-2H-cyclopenta[b]-furan-2-ol, in 20 ml HMPA gelöst, gegeben. Die erhaltene Lösung wurde 15 Minuten bei Raumtemperatur gerührt und dann tropfenweise mit 1 ml Eisessig versetzt, bis die Lösung ... wurde. Das HMPA wurde dann unter Hochvakuum entfernt, der Rückstand mit 50 ml 1 N Natriumhydroxyd versetzt und das Gemisch 18 Stunden gerührt. Der Niederschlag wurde abfiltriert und die wässrige Lösung mit verdünnter ... angesäuert. Das erhaltene Gemisch wurde mit Aether extrahiert, die ätherische Lösung über Natriumsulfat ... und das Lösungsmittel unter vermindertem Druck ...

BAD ORIGINAL

Chromatographie des Rückstandes lieferte 2.4 g nat-11R-Methyl-16R-fluor-15R-[2-tetrahydro-(2H)-pyranyloxy]-9S-hydroxyprosta-5(Z)-ensäure als dickes farbloses Oel; Massenspektrum: m/e 456, 438.

Zu einer rasch gerührten Lösung von 838 mg nat-11R-Methyl-16R-fluor-15R-[2-tetrahydro-(2H)-pyranyloxy]-9S-hydroxyprost-5(Z)-ensäure in 100 ml Aether-Aceton (5:1) wurden bei $0^{o}C$ 0.6 ml Jones-Reagenz gegeben. Nach 10 Minuten wurden einige Tropfen Isopropanol zugesetzt und nach einigen Minuten 1.5 g Natriumbicarbonat und 5 ml Wasser. Sodann wurden 100 ml Aether zugesetzt, das Reaktionsgemisch wurde filtriert und die organische Phase mit gesättigter Natriumchloridlösung gewaschen. Die ätherische Lösung wurde dann getrocknet und das Lösungsmittel unter vermindertem Druck entfernt. Man erhielt rohe nat-11R-Methyl-16R-fluor-15R-[2-tetrahydro-(2H)-pyranyloxy]-9-oxoprost-5(Z)-ensäure.

## Beispiel 7

Zu einer Lösung von 600 mg 11R-Methyl-16R-fluor-15R-hydroxy-9-oxoprost-5(Z)-ensäure in 50 ml Aether/Aceton (5:1 Volumteile) wurde Jones-Reagenz gegeben. Nach Aufarbeitung wie in Beispiel 1 erhielt man 11R-Methyl-16R-fluor-9,15-dioxoprost-5(Z)-ensäure als dickes farbloses Oel. Massenspektrum: m/e 385, 350, 348.

## Beispiel 8

In Analogie zu den Beispielen 6 und 7 wurde aus nat-11R-Methyl-16R-fluor-15S-[2-tetrahydro-(2H)-pyranyloxy]-9S-hydroxyprost-5(Z)-ensäure die nat-11R-Methyl-16R-fluor-15S-hydroxy-9-oxoprost-5(Z)-ensäure, ein dickes farbloses Oel erhalten.
Massenspektrum: m/e 370, 352.

Die Ausgangsverbindung wurde aus 3,3aR,4,5,6,6aS-Hexahydro-4R-[4R-fluor-3S-hydroxy-1(E)-octenyl]-5R-methyl-2H-cyclopenta[b]furan-2-on via 3,3aR,4,5,6,6aS-Hexahydro-4R-[4R-fluor-3S-hydroxyoctanyl]-5R-methyl-2H-cyclopenta[b]furan-2-on und 3,3aR,4,5,6,6aS-Hexahydro-4R-[4R-fluor-3S-(2-tetrahydro-(2H)-pyranyloxy)-octanyl]-5R-methyl-2H-cyclopenta[b]-furan-2-ol hergestellt.

## Beispiel 9

In Analogie zu Beispiel 1 wurde aus nat-11R-Methyl-16,16-difluor-15R-hydroxy-9-oxoprosta-5(Z)-13(E)-diensäure die nat-11R-Methyl-16,16-difluor-9,15-dioxoprosta-5(Z)-13(E)-diensäure erhalten.

## Beispiel 10

In Analogie zu den Beispielen 6 und 7 wurde aus nat-16,16-Dimethyl-9S-hydroxy-15R-[2-tetrahydro-(2H)-pyranyloxy]-prost-5(Z)-ensäure die nat-16,16-Dimethyl-15R-hydroxy-9-oxoprosta-5(Z)-ensäure erhalten.

Die Ausgangsverbindung wurde aus 3,3aR,4,5,6,6aS-Hexahydro-4R-[4,4-dimethyl-3R-hydroxy-1(E)-octenyl]-2H-cyclopenta[b]furan-2-on via 3,3aR,4,5,6,6aS-Hexahydro-4R-(4,4-dimethyl-3R-hydroxyoctanyl)-2H-cyclopenta[b]furan-2-on und 3,3aR,4,5,6,6aS-Hexahydro-4R-[4,4-dimethyl-3R-(2-tetrahydro-(2H)-pyranyloxy)octanyl]-2H-cyclopenta[b]furan-2-ol hergestellt.

## Beispiel 11

In Analogie zu Beispiel 6 und 7 wurde aus [illegible]fluormethyl-16R,11R-dimethyl-15R-[2-tetrahydro-(2H)-pyranyloxy]-9S-hydroxyprost-5(Z)-ensäure die 15R-T[illegible]-16R,11R-dimethyl-15R-hydroxy-9-oxoprost-5(Z)-ensäure erhalten.

BAD ORIGINAL

Die Ausgangsverbindung wurde aus 3,3aR,4,5,6,6aS-Hexahydro-4R-[4R-methyl-4R-trifluormethyl-3R-hydroxy-1(E)-octenyl]-5R-methyl-2H-cyclopenta[b]furan-2-on via 3,3aR,4,5,-6,6aS-Hexahydro-4R-(4R-methyl-4R-trifluormethyl-3R-hydroxy-octanyl)-5R-methyl-2H-cyclopenta[b]furan-2-on und 3,3aR,4,5,-6,6aS-Hexahydro-4R-[4R-methyl-4R-trifluormethyl-3R-[2-tetra-hydro-(2H)-pyranyloxy]-octanyl]-5R-methyl-2H-cyclopenta[b]-furan-2-ol hergestellt.

Beispiel 12

In Analogie zu Beispiel 6 und 7 wurde aus nat-16R-Trifluormethyl-16R-fluor-15R-[2-tetrahydro-(2H)-pyranyloxy]-11R-methyl-9S-hydroxyprost-5(Z)-ensäure die nat-16R-Trifluormethyl-16R-fluor-15R-hydroxy-11R-methyl-9-oxoprost-5(Z)-ensäure erhalten.

Die Ausgangsverbindung wurde aus 3,3aR,4,5,6,6aS-Hexahydro-4R-[4R-trifluormethyl-4R-fluor-3R-hydroxy-1(E)-octenyl]-5R-methyl-2H-cyclopenta[b]furan-2-on via 3,3aR,-4,5,6,6aS-hexahydro-4R-(4R-trifluormethyl-4R-fluor-3R-hydroxy-octanyl)-5R-methyl-2H-cyclopenta[b]furan-2-on und 3,3aR,4,5,6,6aS-Hexahydro-4R-[4R-trifluormethyl-4R-fluor-3R-(2-tetrahydro-(2H)-pyranyloxy)-octanyl]-5R-methyl-2H-cyclopenta[b]furan-2-ol hergestellt.

Beispiel 13

In Analogie zu Beispiel 7 wurde aus nat-16,16-Dimethyl-15R-hydroxy-9-oxoprost-5(Z)-ensäure die nat-16,16-Dimethyl-9,15-dioxoprost-5(Z)-ensäure erhalten.

Beispiel 14

In Analogie zu Beispiel 7 wurde aus nat-16R-Trifluormethyl-16R,11R-dimethyl-15R-hydroxy-9-oxoprost-5(Z)-ensäure die nat-16R-Trifluormethyl-16R,11R-dimethyl-9,15-dioxoprost-5(Z)-ensäure erhalten.

## Beispiel 15

In Analogie zu Beispiel 7 wurde aus nat-16R-Tri-fluormethyl-16R-fluor-15R-hy[illegible] 5(Z)-ensäure die nat-16R-Trifluormetyhl-16R-fluor-[illegible]-methyl-9,15-dioxoprost-5(Z)-ensäure erhalten.

## Beispiel 16

500 mg nat-11R-Methyl-16R-fluor-15R-[illegible] (2H)-pyranyloxy]-9S-hydroxy-prost-5(Z)-ensäure wurden [illegible] 50 ml eines Gemisches von Essigsäure, Wasser und Tetra-hydrofuran (55:30:15 Volumenteile) 18 Stunden [illegible] erwärmt. Danach wurde das Lösungsmittel unter vermindertem Druck entfernt und das als Rückstand verbleibende Oel an einer Silicagel-Säule chromatographiert. Mit [illegible] acetat/Benzol als Elutionsmittel wurden 400 mg [illegible] 11R-Methyl-16R-fluor-9S,15R-dihydroxy-prosta-5(Z)-ensäure als farbloses Oel erhalten.

## Beispiel 17

In Analogie zu Beispiel 16 wurde aus nat-[illegible] methyl-9S-hydroxy-15R-[2-tetrahydro-(2H)-pyranyloxy]-prost-5(Z)-ensäure die nat-16,16-Dimethyl-9S,15R-dihydroxy-prost-5(Z)-ensäure erhalten.

## Beispiel A

Kapseln wurden wie folgt hergestellt:

BAD ORIGINAL

| | | |
|---|---|---|
| nat. 11R-Methyl-16R-fluor-9,15 dioxoprosta-5(Z),13(E)-diensäure | 200 | Gew.-Teile |
| Dikalziumphosphatdihydrat, ungemahlen | 235 | " |
| Maisstärke | 70 | " |
| FD & C Yellow # 5 - Aluminium Lake 25% | 2 | " |
| Durkee Duratex (hydriertes Baumwollsamenöl (voll gesättigt) | 25 | " |
| Kalziumstearat | 3 | " |
| | 535 | Gew.-Teile |

Die oben genannten Bestandteile wurden gründlich gemischt und in Hartgelatinekapseln mit einem Füllgewicht von 350 mg abgefüllt.

## Beispiel B

Tabletten wurden wie folgt hergestellt:

| | |
|---|---|
| nat. 11R-Metyhl-16R-fluor-9,15-dioxoprosta-5(Z)-13(E)-diensäure | 25 mg |
| Dikalziumphosphat Dihydrat, ungemahlen | 175 mg |
| Maisstärke | 24 mg |
| Magnesiumstearat | 1 mg |
| | 225 mg |

Der Wirkstoff und die Maisstärke wurden gemischt und gesiebt. Dieser Prämix wurde dann mit dem Kalziumphosphat und der Hälfte des Magnesiumstearates vermischt, gesiebt und kompaktiert. Das so erhaltene Granulat wurde gesiebt, mit dem restlichen Magnesiumstearat versetzt, und gepresst.

Patentansprüche

1. Prostaglandinderivate der Formel

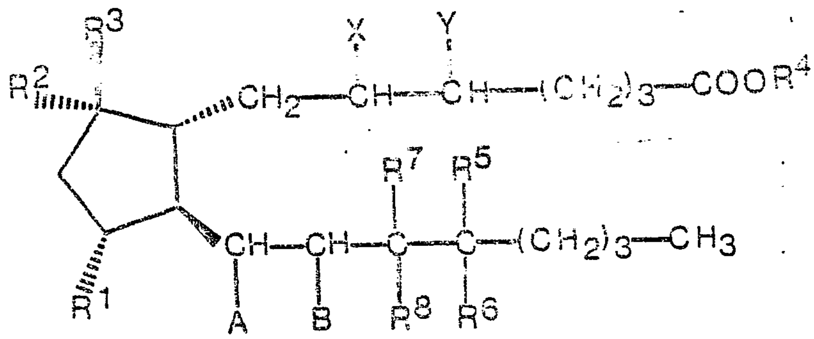

worin $R^1$ Wasserstoff oder Niederalkyl; $R^2$ Hydroxy; $R^3$ Wasserstoff; oder $R^2$ und $R^3$ zusammen Oxo; $R^4$ Wasserstoff oder Niederalkyl; $R^5$ Fluor, Niederalkyl oder Trifluormethyl; $R^6$ Wasserstoff, Fluor oder Niederalkyl; $R^7$ Wasserstoff; $R^8$ Hydroxy; oder $R^7$ und $R^8$ zusammen Oxo; A und B, und X und Y, für sich allein Wasserstoff oder zusammen eine Kohlenstoff-Kohlenstoffbindung bedeuten; mit der Bedingung, dass $R^2$ und $R^3$ zusammen Oxo darstellen, wenn $R^7$ und $R^8$ zusammen Oxo sind;

BAD ORIGINAL

und mit der weiteren Bedingung, dass A und B Wasserstoff darstellen, wenn $R^7$ Wasserstoff ist; und mit der dritten Bedingung, dass X und Y eine Kohlenstoff- Kohlenstoffbindung darstellen, wenn $R^1$ und $R^7$ gleichzeitig Wasserstoff sind;

deren Enantiomere und Racemate.

2. Prostaglandinderivate gemäss Anspruch 1 der Formel

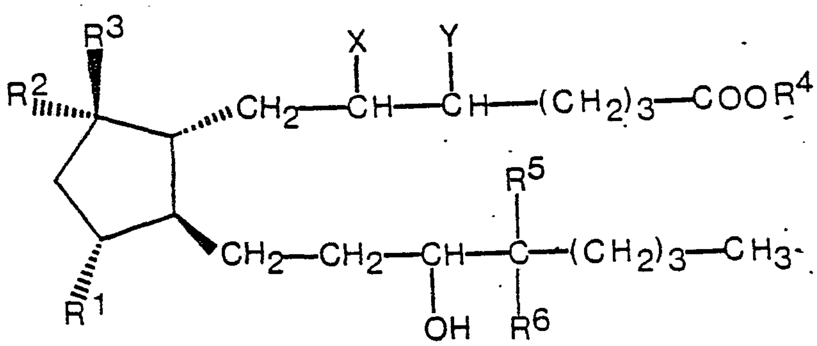

IA

worin $R^1$ bis $R^6$, X und Y die obige Bedeutung haben, mit der Bedingung, dass X und Y eine Kohlenstoff- Kohlenstoffbindung darstellen, wenn $R^1$ Wasserstoff ist.

3. Prostaglandinderivate gemäss Anspruch 1 der Formel

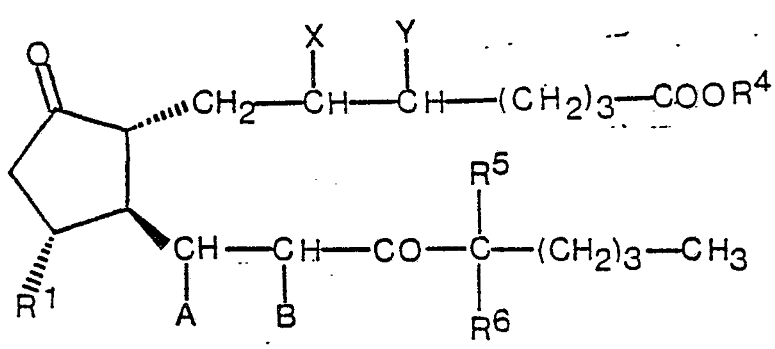

IB

worin $R^1$, $R^4$, $R^5$, $R^6$, A, B, X und Y die obige Bedeutung haben.

4. Verbindungen [illegible] eine Kohlenstoff- Kohlenstoffbindung [illegible]

5. Verbindungen gemäss Anspruch 3, [illegible] Wasserstoff darstellen.

6. Verbindungen gemäss Anspruch 2, worin $R^3$ Wasserstoff und $R^2$ und $R^8$ Hydroxy sind.

7. Nat. 11R-Methyl-16R-fluor-9S,15R-[illegible]-5-(Z)-en-säure.

8. Verbindungen gemäss Anspruch 2, [illegible] Oxo und $R^8$ Hydroxy sind.

9. Nat. 11R-Methyl-16R-fluor-15R-hydroxy-9-[illegible]-5-(Z)-en-säure.

10. Nat. 11R-Methyl-16R-fluor-15S-hydroxy-[illegible]-5(Z)-en-säure.

11. Nat. 16,16-Dimethyl-15R-hydroxy-9-[illegible])-en-säure.

12. Nat. 16R-Trifluormethyl-16R,11R-dimethyl-[illegible]-hydroxy-9-oxoprost-5(Z)-en-säure.

13. Nat. 16R-Trifluormethyl-16R-fluor-15R-[illegible]-11R-methyl-9-oxoprost-5(Z)-en-säure.

14. Nat. 11R-Methyl-16R-fluor-9,15-dioxoprost[illegible]-5(Z),13(E)-dien-säure.

15. Nat. 16R-Fluor-9,15-dioxoprosta-5([illegible]-dien-säure.

16. Nat. 16R-Trifluor[illegible]thyl-9,1[illegible]-13(E)-dien-säure.

BAD ORIGINAL

17. Nat. 11R,16,16-Trimethyl-9,15-dioxoprosta-5(Z),-13(E)-dien-säure.

18. Nat. 16R-Trifluormethyl-16R,11R-dimethyl-9,15-dioxoprost-5-(Z)-en-säure.

19. Nat. 16R-Trifluormethyl-16R-fluor-11R-methyl-9,15-dioxoprost-5(Z)-en-säure.

20. Nat. 11R-Methyl-16R-fluor-9,15-dioxoprost-5(Z)-en-säure.

21. Verfahren zur Herstellung von Prostaglandin-derivaten der Formel

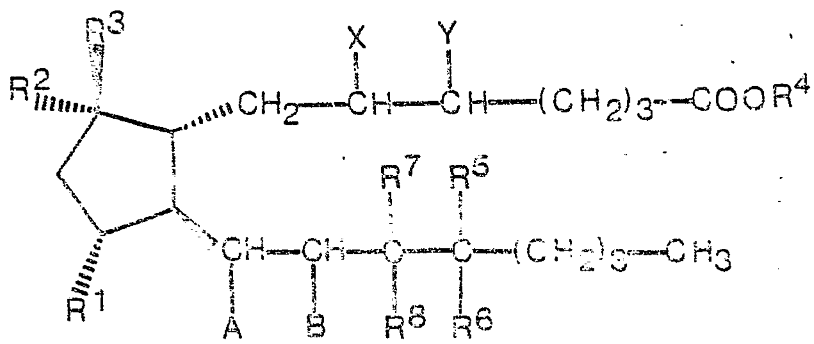

worin $R^1$ Wasserstoff oder Niederalkyl; $R^2$ Hydroxy; $R^3$ Wasserstoff; oder $R^2$ und $R^3$ zusammen Oxo; $R^4$ Wasserstoff oder Niederalkyl; $R^5$ Fluor, Niederalkyl oder Trifluormethyl; $R^6$ Wasserstoff, Fluor oder Niederalkyl; $R^7$ Wasserstoff; $R^8$ Hydroxy; oder $R^7$ und $R^8$ zusammen Oxo; A und B, und X und Y, für sich allein Wasserstoff oder zusammen eine Kohlenstoff-Kohlenstoffbindung bedeuten; mit der Bedingung, dass $R^2$ und $R^3$ zusammen Oxo darstellen, wenn $R^7$ und $R^8$ zusammen Oxo sind; und mit der weiteren Bedingung, dass A und B Wasserstoff darstellen, wenn $R^7$ Wasserstoff ist; und mit der dritten Bedingung, dass X und Y eine Kohlenstoff- Kohlenstoffbindung darstellen, wenn $R^1$ und $R^7$ gleichzeitig Wasserstoff sind;

deren Enantiomeren und Racematen, dadurch gekennzeichnet, dass man

a) eine Verbindung der Formel XI

BAD ORIGINAL

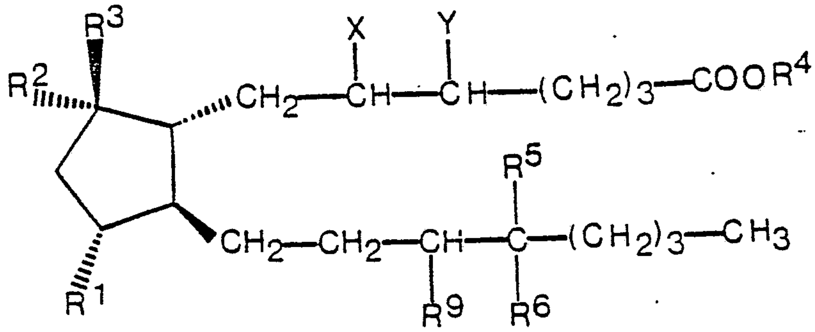

II

worin $R^9$ eine durch eine hydrolysierbare Aether- oder Estergruppe geschützte Hydroxygruppe darstellt und $R^1$ bis $R^6$, X und Y die obige Bedeutung haben

oder ein Enantiomer oder Racemat davon der Hydrolyse der Schutzgruppe $R^9$ unterwirft und gewünschtenfalls eine durch X und Y dargestellte Kohlenstoff- Kohlenstoffbindung hydriert, und/oder eine Hydroxygruppe zur Oxogruppe oxidiert; oder

b) die Hydroxygruppe(n) in einer Verbindung der Formel III

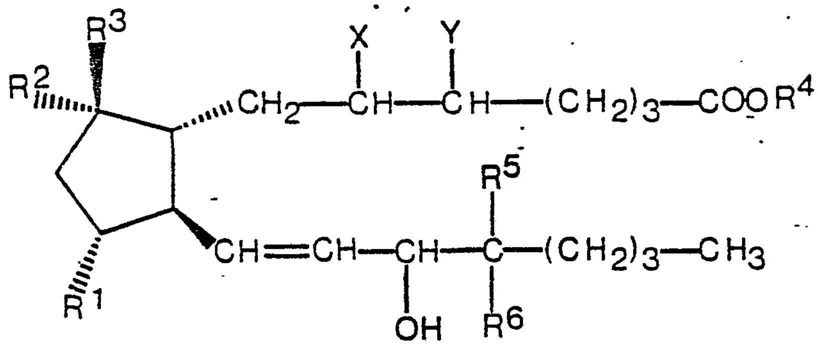

III

worin $R^1$ bis $R^6$, X und Y die obige Bedeutung haben,

oder eines Enantiomers oder Racemats davon zu einer Verbindung der Formel I, worin $R^2$ und $R^3$ bzw. $R^7$ und $R^8$ Oxo darstellen, oxidiert.

22. Verfahren nach Anspruch [illegible], dadurch gekennzeichnet, dass man Verbindungen der Formel

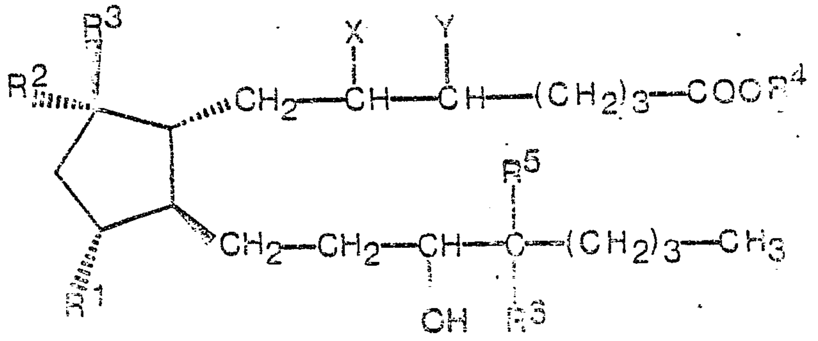

IA

worin $R^1$ bis $R^5$, X und Y die obige Bedeutung haben, mit der Bedingung, dass X und Y eine Kohlenstoff- Kohlenstoffbindung darstellen, wenn $R^1$ Wasserstoff ist,
herstellt.

23. Verfahren nach Anspruch 21, dadurch gekennzeichnet, dass man Verbindungen der Formel

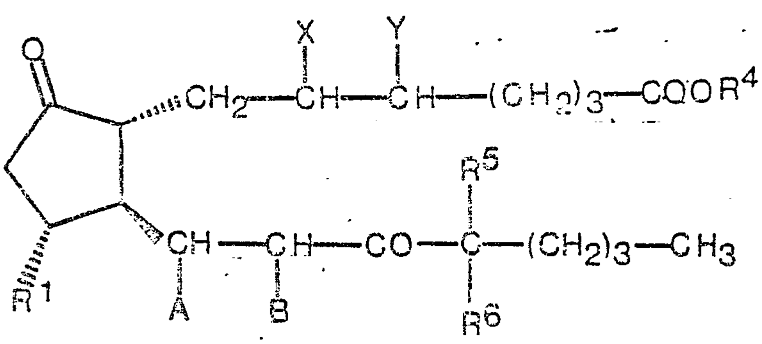

worin $R^1$, $R^4$, $R^5$, $R^5$, A, B, X und Y die obige Bedeutung haben,
herstellt.

BAD ORIGINAL

24. Verfahren nach Anspruch 23, dadurch gekennzeichnet, dass man Verbindungen der Formel IB herstellt, in denen A und B eine Kohlenstoff- Kohlenstoffbindung darstellen.

25. Verfahren nach Anspruch 23, dadurch gekennzeichnet, dass man Verbindungen der Formel IB herstellt, in denen A und B Wasserstoff darstellen.

26. Verfahren nach Anspruch 21, dadurch gekennzeichnet, dass man nat. 11R-Methyl-16R-fluor-9,15-dioxoprosta(Z)-5-(E)-13-dien-säure herstellt.

27. Verfahren nach Anspruch 21, dadurch gekennzeichnet, dass man nat. 11R-Methyl-16R-fluor-15R-hydroxy-9-oxo-prost-(Z)-5-en-säure herstellt.

28. Verfahren nach Anspruch 21, dadurch gekennzeichnet, dass man nat. 11R-Methyl-16R-fluor-9,15-dioxo-prost-(Z)-5-en-säure herstellt.

29. Pharmazeutische Präparate, gekennzeichnet durch einen Gehalt an einem Prostaglandinderivat der Formel I gemäss Definition in Anspruch 1.

30. Verfahren zur Herstellung pharmazeutischer Präparate, dadurch gekennzeichnet, dass man ein Prostaglandinderivat der Formel I gemäss Definition in Anspruch 1 mit nicht-toxischen, inerten, physiologisch verträglichen festen oder flüssigen, an sich in solchen Präparaten üblichen Trägerstoffen mischt.

31. Zwischenpr[illegible] [illegible]bindungen
gemäß Anspruch [illegible] Formel

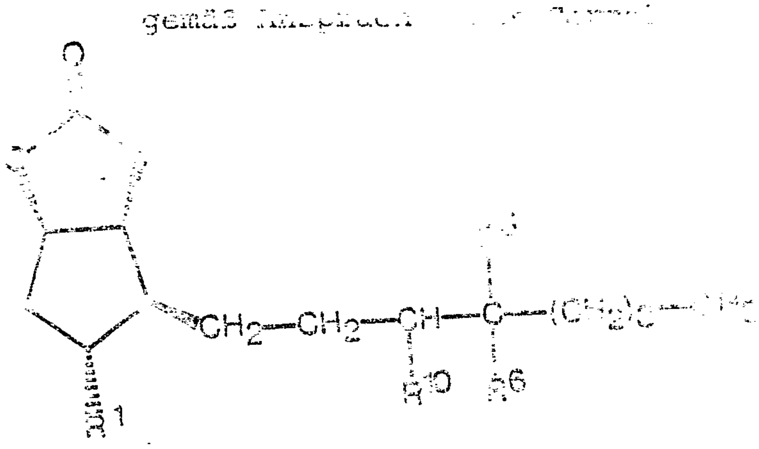

worin $R^1$ Wasserstoff oder Niederalkyl, $R^5$ Fluor, Niederalkyl oder Trifluormethyl, $R^6$ Wasserstoff, Fluor oder Niederalkyl, und $R^{10}$ gegebenenfalls geschütztes Hydroxy ist.

32. Zwischenprodukte zur Herstellung der Verbindungen gemäß Anspruch 1 der Formel

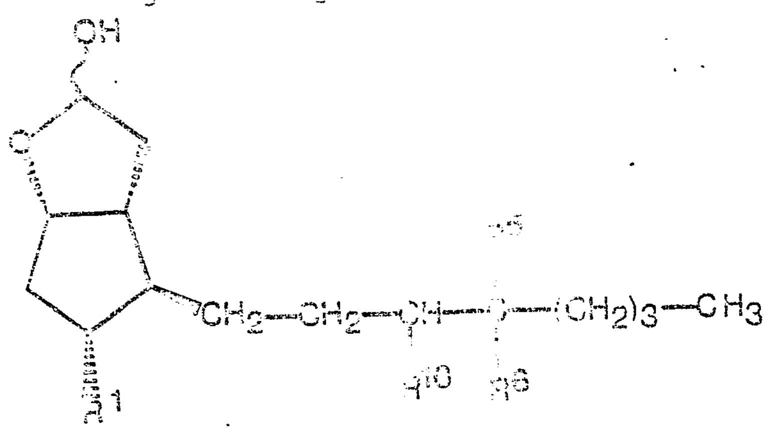

worin $R^1$ Wasserstoff oder Niederalkyl, $R^5$ Fluor, Niederalkyl oder Trifluormethyl, $R^6$ Wasserstoff, Fluor oder Niederalkyl, und $R^{10}$ gegebenenfalls geschütztes Hydroxy ist.

33. Zwischenprodukte [illegible] Herstellung [illegible]
gemäß Anspruch [illegible] Formel

BAD ORIGINAL

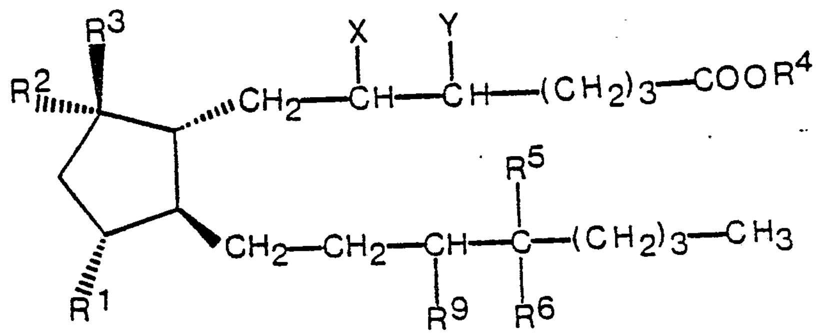

II

worin $R^9$ geschütztes Hydroxy darstellt und $R^1$ bis $R^6$, X und Y wie in Anspruch 1 definiert sind, wobei X und Y eine C-C-Bindung darstellen, wenn $R^1$ Wasserstoff ist.

Europäisches Patentamt

# EUROPÄISCHER RECHERCHENBERICHT

Nummer der Anmeldung
EP 78 10 0303

0000207

## EINSCHLÄGIGE DOKUMENTE

| Kategorie | Kennzeichnung des Dokuments mit Angabe, soweit erforderlich, der maßgeblichen Teile | betrifft Anspruch |
| --- | --- | --- |
| X | US - B - 491 711 (WENLING KAO et al.)<br>* Ansprüche 2-7, 9-11,13; Spalte 1, Zeilen 10 bis 25 *<br>-- | 1,3-5, 29,30, 21-23, 33 |
| X | FR - 2 301 242 (C.V.GRUDZINSKAS et al.)<br>* Ansprüche 1,24-26 *<br>-- | 1-30,33 |
| X | FR - 2 322 593 (G.L.BUNDY et al.)<br>* Ansprüche 1-10; Seiten 23-67 *<br>---- | 1-11, 21,22, 27,29-33 |

KLASSIFIKATION DER ANMELDUNG (Int.Cl.²)

C 07 C 177/00

RECHERCHIERTE SACHGEBIETE (Int. Cl.²)

C 07 C 177/00

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
| --- | --- | --- |
| Den Haag | 05-10-1978 | BERTE |

BAD ORIGINAL